# EUROPÄISCHE PATENTSCHRIFT

(11) **EP 0 374 935 B1**
(45) Veröffentlichungstag und Bekanntmachung des Hinweises auf die Patenterteilung: **30.06.1993**
(21) Anmeldenummer: 89123731.5
(22) Anmeldetag: 22.12.1989
(51) Int. Cl.: A61K 33/42, A61K 33/10, A61K 33/06

(54) **Präparat zum Vorbeugen gegen Knochenschwund und zur Behandlung von Knochenschwund**
Preparation for preventing osteoporosis and for treating osteoporosis
Préparation pour prévenir l'ostéoporose et pour traiter l'ostéoporose

(30) Priorität: 22.12.1988 HU 657388
(43) Veröffentlichungstag der Anmeldung: 27.06.1990
(73) Patentinhaber: Márkus, Jozsef, Dr., H-1028 Budapest (HU)
(72) Erfinder: Márkus, Jozsef, Dr., H-1028 Budapest (HU)
(74) Vertreter: Hansen, Bernd, Dr. Dipl.-Chem.

(56) Entgegenhaltungen:
- DE-A- 2 226 273
- UNLISTED DRUGS, Band 36, Nr. 5, Mai 1984, Seite 82, Chatham, New Jersey, US

## Beschreibung

Die Erfindung betrifft ein Präparat zum Vorbeugen gegen Knochenschwund (Osteoporose) und zur Behandlung von Knochenschwund.

Knochenschwund wird im allgemeinen als eine Begleiterscheinung des Alterns betrachtet, was ein doppelter Fehler ist, denn erstens können auch ganz junge Menschen an Knochenschwund leiden, und zweitens hat nicht jeder alte Mensch Osteoporose. Einer anderen allgemein verbreiteten Ansicht nach wird der Knochenschwund (wenigstens bei Frauen) dadurch verursacht, daß mit der Klimax die Produktion der Geschlechtshormone aufhört. Auf beide Ansichten wird später noch eingegangen.

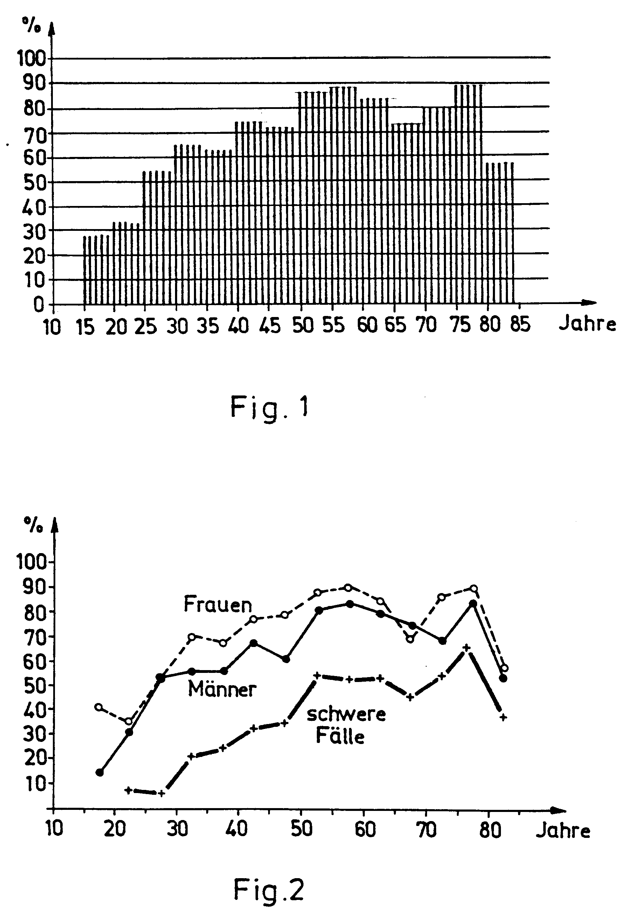

Der Knochenschwund macht sich als Wirbelsäulen- und Gelenkschmerz bemerkbar. Auch der überwiegende Teil der rheumatologischen Beschwerden ist auf diese Ursache zurückzuführen. In Fig. 1 ist die Häufigkeit des Knochenschwundes als Funktion des Lebensalters abgebildet. Die Daten wurden durch Fragebogenbefragung von 1654 ohne Auswahlkriterien befragten Personen beiderlei Geschlechts zwischen 15 und 84 Jahren erhalten und mittels Computer aufgearbeitet. Dabei ergab sich unter anderem, daß in Ungarn 77,8 % der Frauen und 66,7 % der Männer, d.h. insgesamt 73,1 % der Bevölkerung Beschwerden haben, die auf Knochenschwund schließen lassen. Das sind mehr als 6 Millionen Menschen. Fig. 1 zeigt folgendes:
1) Knochenschwund ist keine Alterskrankheit, sondern kann schon im Jugendalter beginnen;
2) der als Funktion des Lebensalters kontinuierliche Anstieg der Kurve läßt eher eine Art Sättigungsprozeß, einen sich akkumulierendon Grund erkennen;
3) die oben erwähnte Klimaxtheorie kann nicht zutreffend sein, denn dann müßte sich bei etwa 50 Jahren ein sprungartiger Anstieg zeigen. Fig. 2 zeigt die Häufigkeit des Knochenschwundes getrennt nach Geschlechtern sowie die Häufigkeit schwerer Fälle als Funktion des Alters. Auch hier ist bei den etwa 50 jährigen Frauen kein scharfes Maximum zu beobachten.

Die durch Osteopose verursachten Beschwerden können sehr unterschiedlich sein, und die meisten Menschen sind sich über den Grund ihrer Beschwerden nicht im klaren. Sie verspüren ein Stechen in der Schulter, Kribbeln in den Fingern, ihnen tut das Genick weh, das Kreuz, die Knie. Von den Frauen über Vierzig sind 19,5 % beschwerdefrei, die Hälfte klagt über Schmerzen im Knie, 40 % haben Hüftgelenksbeschwerden. Bei 29 % der Mädchen zwischen 15 und 19 zeigt die Wirbelsäule eine Krümmung nach der Seite. In Ungarn werden jährlich etwa 40 000 Menschen wegen durch Knochenschwund verursachter"unheilbarer" Leiden in den Invalidenstand versetzt. Der Schenkelhalsbruch ist im Alter eine der häufigsten mittelbaren Todesursachen. Der gekrümmte Rücken, die Schrumpfung der Körpergröße um 8-10 cm, Lumbago, Ischias sind ebenfalls Erscheinungsbilder des Knochenschwundes.

Im folgenden wird ein Überblick über die bisher übliche Therapie der Osteoporose gegeben.

Wie bereits erwähnt, hatte die zufällige Erscheinung, daß etwa 15 % der in der Menopause befindlichen Frauen an durch Knochenschwund verursachten Beschwerden leiden, die Aufmerksamkeit schon früh auf die Rolle des Östrons gelenkt; Therapie durch Östrongaben zeigte kein eindeutiges Ergebnis. Die Tatsache, daß in den Knochen keine Östronrezeptoren vorhanden sind, stellte die ganze Therapie stark in Frage. Erwähnt werden muß auch, daß die anhaltende Östrontherapie zahlreiche unerwänschte Nachteile und Nebenwirkungen hat (Hemmung der Kollagensynthese, Gebärmutterkrebs, Brustkrebs.) Dies und die Tatsache, daß ein wirklich durchschlagender therapeutischer Erfolg ausblieb, haben in der Östrontherapie eher zu einer Verunsicherung geführt.

Eine andere Therapierichtung geht von der Rolle des Calcitonins aus. Calcitonin ist ein in der Schilddrüse gebildetes Polypeptid-Hormon, seine Aufgabe ist die Regulierung des Ca-Spiegels im Blut. Als Calcitonin-Präparat ist unter der Bezeichnung MIACALCIC ein synthetisches Lachscalcitonin auf dem Markt, das für sich allein schmerzstillend wirkt. Die Behandlung beruht auf irrigen Voraussetzungen, denn der primär osteoporosekranke Patient ist unter dem Aspekt der regulierenden Hormnfunktion des Kalkhaushaltes gesund, seine Krankheit ist vielmehr der Mangel an Calcium.

Auch mit fluorhaltigen Präparaten (KOREBEREON) wurde versucht, im Zustand der an Knochenschwund Leidenden eine Besserung zu erreichen. Das Fluor wurde als ständige Komponente der Knochen und Zähne schon 1805 erwähnt; daß es jedoch essentiell und unentbehrlich wäre, konnte bisher auch mit über Generationen dauernden Tierversuchen nicht erwiesen werden. Das Fluor ist von allen Elementen das negativste und deshalb sehr reaktiv; es wird überall eingebaut, auch in die Knochen. Sein Natriumsalz wird zu über 90 % resorbiert, und weil das Fluor sehr langsam ausgeschieden wird, akkumuliert es sich vor allem in den Zähnen und in den Knochen. Der in über die normale Menge (0,1 %) hinausgehender Menge Fluor enthaltende Knochen ist brüchig. Auch greift das Fluor in physiologische Prozesse ein (zum Beispiel wird durch die Bildung von Fluoracetat und daran anschließend Fluoracetylcoenzym A das Enzym Akonitsäurehydratase gehemmt), und kann als Jodantagonist eine indirekte Rolle in der Entstehung von Struma spielen.

In der Therapie des Knochenschwundes werden auch kalkhaltige Präparate verwandet. Das FONO-Granulat besteht aus 50 % Tricalciumphosphat, 20 % Calciumcarbonat und 30 % Zucker. Die als tägliche Dosis vorgeschriebene Menge von 3x1 gestrichenen Kaffeelöffel voll enthält etwa 1,75 g Calcium und 0,486 g Phosphor; das ist erstens zur Linderung des Knochenschwundes zu wenig, zum anderen führt das zusammen mit dem Calcium und Phosphor, der in der täglichen Nahrung enthalten ist, zu einem vom optimalen abweichenden Ca:P Verhältnis. (Auf das Ca:P Verhältnis als auf einen Faktor von essentieller Wichtigkeit wird noch zurückgekommen.)
Die CALDEA Tablette ist ebenfalls ein kalkhaltiges Präparat, welches außer Vitamin D in jeder Tablette 200 mg Calciumlactat und 400 mg Calciumphosphat enthält. Auch bei einer Gabe von täglich 2x3 Tabletten werden dem Organismus täglich nur 1400 mg Ca-Nachschub zugeführt, das ist zu wenig für eine Besserung des Knochenschwundes. Wird die Tablette in der vorgeschriebenen Weise genommen, so erhält der Organismus täglich das 10fache des Tagesbedarfes an Vitamin D.

Aus DE-A-2 226 273 sind pharmazeutische Präparate zur Behandlung der Osteoporose bekannt, die dadurch gekennzeichnet sind, daß sie als essentielle Wirkstoffe a) Glucosamin und/oder pharmazeutisch annehmbare Salze davon und b) ein oder mehrere pharmazeutisch annehmbare Calciumsalze enthalten. Die Kombination mit Glucosamin oder dessen Derivaten soll einen gesteigerten Anstieg der Calciumresorption im Körper bewirken.

Schließlich umfaßt die Therapie der auf Knochenschwund zurückzuführenden Leiden auch physikotherapeutische Maßnahmen und Heilbäder. Diese Institutionen, Kliniken und Heilbäder führen eine sorgfältige und vielschichtige Arbeit aus, die aber trotzdem nur in symptomatischer Behandlung besteht: Lösen von Kontrakturen, Muskelkrämpfen, Schmerzlinderung usw; die Wurzel des Übels wird nicht berührt, deshalb ist die Erleichterung auch nur vorübergehend.

Der Ernst der Lage wird von der Überfülltheit der rheumatologischen Fachordinationen, der gedrückten Stimmung der wartendan Patienten, dem riesigen Anstieg des Verbrauchs an Schmerztabletten wiedergespiegelt.

Die Erfindung beruht auf der Erkenntnis, daß die Osteoporose nicht hormonellen Ursprungs ist, nicht durch Arzneimittel oder Physikotherapie geheilt werden kann und vor allen Dingen nicht unheilbar ist, sondern eine Folge unrichtiger Ernährung darstellt und als solche durch entsprechende Ergänzung der Ernährung geheilt werden kann.

Wie bekannt ist, ist in den Knochen das Kalk-Phosphor-Verhältnis 2:1. Es wäre deshalb wünschenswert, wenn dieses Verhältnis auch in den Nahrungsmitteln bestehen würde. Das verhält sich jedoch nicht so, sondern die meisten Speisen und Lebensmittel enthalten bis zu 10 mal so viel Phosphor, und der Überschuß des Phosphors wird vom Organismus ausgeschieden, und zwar in Form von Calciumphosphat. Die zur Bildung des Phosphats benötigten Calciumionen werden vom Calciumspiegel des Blutserums (seinem gelösten Calciumgehalt) geliefert, und das zur Aufrechterhaltung des Calciumspiegels erforderliche Calcium wird den Knochen entzogen. (Der Prozeß ist hier etwas vereinfacht dargestellt, denn einesteils hat das Calcium im Organismus noch zahlreiche andere vielschichtige Aufgaben, und zum anderen ist die hormonelle Regelung des Kalkhaushaltes ein sehr komplizierter Prozeß; für das Verständnis der Erfindung ist die oben beschriebene Modellvorstellung jedoch völlig ausreichend.)
Das Calcium-Phosphor-Verhältnis ist in den meisten Speisen ausgesprochen ungünstig. In der folgenden Tabelle ist für einige Lebensmittel das Ca/P-Verhältnis angegeben.

| Lebensmittel | Ca/P-Verhältnis |
|---|---|
| Wintersalami | 0,09 |
| Kakac | 0,2 |
| Nüsse, Mandeln | 0,5 |
| Eigelb | 0,14 |
| ganzes Ei, ohne Schale | 0,24 |
| Fleisch ohne Knochen | 0,04-0,1 |
| Aufschnitt | 0,04-0,29 |
| Brot, Gebäck | 0,17-0,3 |
| Hülsenfrüchte | 0,12-0,24 |
| Mohn | 1,1 |
| Eßkastanien | 1,2 |
| Schafsquark | 1,5-2,0 |
| Schmelzkäse | 0,45-0,5 |
| Kuhquark | 0,3-0,5 |

Unter dem Aspekt des Knochenschwundes ist der gefährlichste Faktor das Fleisch, weil der Mensch das Fleisch ohne Knochen ißt. Die Raubtiere fressen nur Fleisch und leiden trotzdem nicht an Knochenschwund, weil sie zusammen mit dem Fleisch auch die weichen Knochen fressen. Zahlreiche Mangelkrankheiten werden für gewöhnlich dem hohen Verarbeitungsgrad der modernen Lebensmittel zugeschrieben, für den vorliegenden Fall ist das jedoch nicht zutreffend, denn sogar aus einem siebentausend Jahre alten Grab in Peru kamen - neben von Fleischkonsum zeugenden Haushaltsgerätschaften - Skelette mit Anzeichen von Knochenschwund zum Vorschein. Die Menschheit legte den Grundstein der Osteoporose zusammen mit dem der Zivilisation, als sie zum Verzehr von Fleisch ohne Knochen überging.

Wie die Tabelle zeigt, gewährleisten die Milchprodukte noch eine verhältnismäßig gute Calciumversorgung, im Schafsquark ist das Ca/P-Verhältnis zufriedenstellend, aber so viel Schafsquark, wie zur Wiederherstellung bereits angegriffener Knochen erforderlich wäre, ißt niemand. Es ist sehr zu bedauern, daß bei der Herstellung von Schmelzkäse als Verflüssigungssalze Phosphorsalze verwendet werden, weil dadurch das Ca:P-Verhältnis sehr verschlechtert wird. Der Kuhquark ist auch nicht besser, denn er wird durch Säurefällung hergestellt, die Milchsäure bindet das Calcium, das auf diese Weise in der Molke bleibt.

Dem Organismus des insbesondere Fleisch essenden, wenig pflanzliche Produkte beziehungsweise Milch und Käse konsumierenden Menschen wird eine große Menge überflüssigen Phosphors zugeführt, und das zu seiner Entfernung erforderliche Calcium wird den Knochen entzogen. Der Knochenschwund ist im allgemeinen 30-40 %ig, in schweren Fällen 50 %ig, und selbst 60 % sind nicht selten. Der Phosphor wird als Calciumphosphat mit dem Stuhl ausgeschieden, wodurch dem Organismus täglich 20-40 mg, häufig auch mehr Kalk verlorengehen. Dadurch wird der Verlauf des Diagramms gemäß Figur 1 verständlich: der Verlust von 40 mg akkumuliert sich von Jahr zu Jahr, deswegen ist im Alter die Osteoporose verbreiteter.

Wie aus der Tabelle mit den Ca/P-Verhältnissen hervorgeht, ist es unmöglich, aus den üblichen Nahrungsmitteln eine Speisefolge zusammenzustellen die keinen Knochenschwund verursacht. Die daraus ableitbare Folgerung ist klar: wenn die erforderliche Calciummenge nicht mit der Nahrung zugeführt werden kann, so muß sie dem Organismus zusätzlich zugeführt werden.

Es ist nicht verständlich, warum die Humanheilkunde diese einfache Schlußfolgerung nicht zu ziehen vermochte, um so weniger, als daß auf veterinärmedizinischem Gebiet Bedeutung und Auswirkungen des Verhältnisses bzw. des Fehlens von Calcium, Magnesium, Phosphor seit annähernde 80 Jahren erforscht werden (Oszkár Wellmann: Vizsgálatok a Ca , Mg P forgalmáról éhező állatokban /= Untersuchungen über den Ca-, Mg- und P-Umsatz an hungernden Tieren/; veterinärmedizinische Dissertation, Budapest 1907). Im großen Maßstab gehaltene Schweine und Geflügel erhalten seit 50 Jahren zusätzlich Kalk. Die in veterinärmedizinischer Hinsicht ausgearbeiteten und dokumentierten, statistisch aufbereiteten Ergebnisse zahlloser Experimente könnten sicher auch der Humanheilkunde Ansatzpunkte bieten, ist doch das Schwein ein Allesfresser genau wie der Mensch, und die chemischen Prozesse, die zur Resorption, der Ausscheidung, dem Einbau von Calcium und Phosphor führen, laufen im Menschen auf die gleiche Weise ab wie im Tier.

Im Gegensatz zu zahlreichen Spurenelementen und sonstigen essentiellen Stoffen kann für den Calciumbedarf keine fixe Zahl angegeben werden (mg/kgxTag), weil das jeweils von der Phosphormenge abhängt. Aus dem durchschnittlich 1,9 % betragenden Phosphorgehalt der Speiseeiweiße und dem durchschnittlichen Eiweißkonsum eines Menschen läßt sich errechnen, daß die Phosphoraufnahme bei Frauen etwa 1500 mg, bei Männern etwa 1900 mg beträgt, während der Calciumkonsum bei beiden Geschlechtern lediglich bei etwa 500 mg liegt. Zur Abführung dieser Phosphormenge werden (wenn der Kalkbestand der Knochensubstanz nicht angegriffen werden soll) 2000 mg Ca (das entspricht 5 g Calciumcarbonat) gebraucht; wenn die Knochen schon geschädigt sind, muß die Menge noch erhöht werden.

Es wurde nun gefunden, daß ein feingemahlenes Gemisch aus Calciumcarbonat, Magnesiumcarbonat und Calciumhydrogenphosphat zum Vorbeugen gegen und Behandeln von Knochenschwund geeignet ist . Gegenstand der Erfindung ist demnach ein Präparat zur Behandlung von Knochenschwund. Für das Präparat ist kennzeichnend, daß es
20-45 % Kalksteinmehl,
10-20 % Dolomitmehl.
30-60 % Calciumhydrogenphosphat,
gegebenenfalls höchstens 6 % Mikroelementprämix und gegebenenfalls höchstens 5 % an sich bekannte Zusatzstoffe enthält.
Die Erfindung betrifft ferner ein Präparat zum Vorbeugen gegen Knochenschwund. Für dieses ist kennzeichnend, daß es
40-60 % Kalksteinmehl und
40-60 % Dolomitmehl und
0-6 % Mikroelementprämix
enthält. Die zuerst angegebene Zusammensetzung wird bei bereits bestehendem Knochenschwund in einer Menge von 3x1 Kaffeelöffel täglich eingenommen. Die zweite Zusammensetzung (präventives Mittel) wird zur Aufrechterhaltung des bestehenden Zustandes in einer Menge von täglich etwa 1 Kaffeelöffel eingenommen; die zur Resorption und dem Einbau in die Knochen erforderlichen Vitamine müssen parallel dazu ebenfalls regelmäßig eingenommen werden. Der Vitaminbedarf kann durch täglich 2x1 Tabletten Polyvitaplex 8 (Vitamin A und D), täglich 3x1 Dragee Vitamin C und alle 3 bis 4 Tage eingenommen) eine Kapsel Vitamin E gedeckt werden. Die Vitamine können dem Kalkpulver auch zugemischt werden. In diesem Fall sind auf 1 kg Pulvergemisch 20 000-60 000 IU Vitamin D, 50 000-250 000 IU Vitamin A und 0-1000 IU Vitamin E zu rechnen.

Wie schon aus der angegebenen Dosierung hervorgeht, muß das Präparat in relativ beträchtlichen Mengen eingenommen werden. Eine Therapie von 100 Tagen zum Beispiel kann 2 kg Kalkpulver erfordern. Deswegen und infolge der allgemeinen Verbreitung des Knochenschwundes muß das Kalkpulver in riesigen Mengen bereitgestellt werden. Aus diesem Grund wurde auf die Verwendung analysenreiner Ausgangsstoffe bzw. auf in den Arzneimittelbüchern vorgeschriebene Qualität bewußt verzichtet. Kalkstein und Dolomit stehen in unbegrenzter Menge und billig zur Verfügung, sie müssen nur - vorzugsweise in einer Prill-Mühle - so lange gemahlen werden, bis mehr als 90 % der Teilchen eine Größe von unter 5 Mikron aufweisen.

Der Pulvermischung kann gewünschtenfalls ein Prämix aus Spurenelementen zugesetzt warden. Als Enzymkomponenten und Enzymaktivatoren haben die Mikroelemente für die Ungestörtheit der physiologischen Prozesse und die Bewahrung der Gesundheit große Bedeutung. Der Mikroelementbedarf von Nutztieren wird schon seit einem Vierteljahrhundert aus Milligramm genau dem Futter zugesetzt, nur eben auf den Menschen erstreckt sich diese Fürsorge nicht. Der Mikroelementprämix wird mit Kalkpulver als Streckmittel so zubereitet , daß der tägliche Bedarf von 15 mg Fe, 3 mg Cu, 5 mg Mn, 15 mg Zn, 10 mg B, 0,14 mg J und 0,1 mg Se gedeckt wird.

Das Kalkpulver Mann gewünschtenfalls bis zu 5 % an sich bekannte Zusatzstoffe enthalten. Als solche kommen unterschiedliche Aromastoffe, Zucker, Kakao und Granulierhilfen (Polyvinylpyrrolidon) in Betracht. Das Kalkpulverkann gewünschtenfalls tablettiert, in Kapseln gefüllt oder granuliert warden. Diese Formulierungen sind nur dann angebracht, wenn die Einnahme auf andere Weise nicht möglich ist, denn auf jeden Fall wird das feine, unformulierte Pulver am besten resorbiert.

Die Herstellung des erfindungsgemäßen Präparates wird mit Hilfe der folgenden Beispiele näher erläutert.

### Beispiel 1

### Zusammensetzung:

330 g Kalksteinmehl
160 g Dolomitmehl
490 g Calcium-hydrogen-phosphat
20 g Mikroelementprämix
90 % der Teilchen sind kleiner als 5 Mikron.

| Zusammensetzung des Mikroelementprämixes | |
|---|---|
| Eisenfumarat | 11,760 g |
| Kupfer(II)sulfat | 2,210 g |
| Mangansulfat | 3,930 g |
| ZnO | 4,690 g |
| Calciumjodat | 0,060 g |
| Natriumselenit | 0,060 g |
| Natriumborat | 21,740 g |
| Kalksteinmehl | 55,550 g |
| | 100,000 g |

Diese Zusammensetzung ist zur behandlung bereits ausgebildeter Osteoporose geeignet. An den ersten zwei Tagen beträgt die Dosis 1 Teelöffel, an den beiden folgenden Tagen 2x1 Teelöffel, und ab dem fünften Tag 3x1 gehäufter Teelöffel (6,5-7 g), einzunehmen vor den Hauptmahlzeiten, mit ein paar Schlucken Flüssigkeit (gesüßter Tee, Milch, Milchkaffee, Kakao, Fruchtsaft usw.). Nach den ersten 80 Tagen kann der Löffel Kalkpulver vor dem Mittagessen entfallen, die restliche Zeit der therapeutischen Behandlung reichen täglich 2 Teelöffel. Unter Berücksichtigung dessen, daß die Knochensubstanz etwa alle 1/2 Jahr ausgewechselt (umgebaut) wird, ist die Therapie ebenfalls 1/2 Jahr. Bei Schwerkranken und bei Personen über 60 Jahren kann dieser Zeitraum um 2-3 Monate verlängert werden.

### Beispiel 2

### Zusammensetzung:

60 % Kalksteinmehl
34 % Dolomitmehl
6 % Prämix gemäß Beispiel 1
Das Pulvergemisch mit der angegebenen Zusammensetzung ist zum Vorbeugen gegen Knochenschwund geeignet.Es enthält keinen Phosphor, sondern nur das zur Entfernung des mit den Nahrungsmitteln aufgenommenen überflüssigen Phosphors erforderliche Calcium und Magnesium. Empfohlen wird eine Dosis von täglich 1 Teelöffel (etwa 7 g); das ist ausreichend, um den Phosphor ohne Schädigung der Knochensubstanz aus dem Organismus zu entfernen.

Anwendungsgebiete der Therapie: Osteoporose, Spondylose, Dislopatie, Ostreoarthrose, Rachitis, Osteomalacie, Algoneuro dystrophie), Knochenbrüche, Knochenoperationen, Kompensation der Knochenschwund verursachenden Dauerbehandlung mit Steroiden, Deckung des angestiegenen Calciumbedarfes im letzten Drittel der Schwangerschaft und während des Stillens.

Rheumatische und sonstige Gelenkschmerzen verschwinden bereits nach wenigen Wochen, die Knochensubstanz erneuert sich, wird aufgefüllt, und wenn die Porosität der Knochen bis auf den physiologischen Wert von 24-25 % gesunken ist, beginnt auch die Regeneration des Knorpels.

Während der Kur kann Stuhlverstopfung eintreten. Durch Cellulosefasern enthaltende Speisen, Gemüse, Kleie kann das kompensiert werden. Als Nebenwirkung ist zu erwähnen, daß das Präparat die Resorption von Anthracyclinen verlangsamt; während der Behandlung mit einem Antibiotikum der Anthracyclinreihe ist es deshalb zweckmäßig, die Kur zu unterbrechen.

Im folgenden werden einige Krankenfälle, die Behandlung und das Ergebnis geschildert.

N.N., eine 60jährige Frau, war so unglücklich gefallen, daß ihr 12. Wirbel brach. Das war mit starken Schmerzen verbunden. In 8 verschiedenen Behandlungsstellen konnte man ihr nicht helfen. Sie begann die Kur mit täglich 6 g Kalkpulver-Komposition und viel Eiweiß. Nach vier Wochen konnte sie ohne Unterstützung Kniebeugen machen.

P.I., eine 61jährige Kranke, hatte seit ihrer Kindheit eine Wirbelsäulenkrümmung, die man bemerkte, als das Kind 7 Jahre alt war. Mit 10 Jahren bekam sie ein Korsett, das sie bis zum 18. Lebensjahr trug. Sie schwamm und turnte viel, bis zu ihrem 55. Lebensjahr war sie beschwerdenfrei. 1982 suchte sie wegen Kreuzschmerzen einen Rheumaarzt auf. Auf der Röntgenaufnahme wurde hochgradige convex-lumbale Scoliose nach links festgestellt; gegen die Schmerzen bekam sie Novokain, wurde auch in Heilbädern, durch orthopädisches Turnen behandelt, massiert usw. Trotzdem hatte sie dauernd mehr oder weniger Schmerzen. Sie machte dann vorschriftsmäßig die Kalkpulverkur,und schon nach 2 Monaten hatte sie keinerlei Schmerzen mehr. Gegenwärtig nimmt sie das vorbeugende Kalkgemisch ein, täglich einen Löffel; sie ist mit ihrem Zustand zufrieden.

H.H., ein 24jähriger Sportler, wandte sich wegen plötzlich eingetretener Knieschmerzen an den Arzt. Festgestellt wurde eine hochgradige Abnutzung, eine Operation wurde empfohlen, das Karate-Training verboten. Statt sich operieren zu lassen, begann der junge Mann mit der Kalkpulverkur. Nach zwei Wochen hatte er keine Schmerzen mehr. Er setzte die Kur noch 3 Monate lang fort. Heute ist er völlig beschwerdenfrei, das Karate-Training hat er wieder aufgenommen.

G.I., eine 60jährige Kranke, wurde im Dezember 1986 wegen seit Monaten anhaltenden, beinahe unerträglichen Schmerzen in beiden Hüftgelenken (laufen konnte sie kaum noch, und schlafen nur mit schmerzstillenden Mitteln) ins Margit-Krankenhaus eingewiesen. Dort wurde sie geröntgt, in beiden Hüftgelenken wurde starke Knorpelabnutzung (Coxarthrose) festgestellt und die Kranke zur orthopädischen Chirurgie überwiesen. Sie versuchte es lieber mit der Kalkpulverkur, Drei Monate lang nahm sie das Kalkpulver ein, ab der 5. Woche wurden die Schmerzen schwächer, und am Ende der Kur war sie völlig beschwerdenfrei. Ihre funktionelle Bewegung, ihr Gang sind beinahe vollkommen. Sie kann ohne Anstrengung auch schnell laufen und Treppen steigen.

Frau J.J. wurde wegen Kreuzschmerzen geröntgt, man stellte starken Knochenschwund fest. Sie bekam folgende Behandlung: Voltaren täglich 3x1, Naprosin 10 Tage lang 2x1, 26 Tabletten Nerobol, 30 Kapseln Biarison (3x1 täglich), 100 Tabletten Ibuprofen (3x1 täglich), schmerzstillende Mittel (analgetisch wirkende Suppositorien, Algopyrin als Tabletten und als Injektion) vom 26. Mai bis zum 23. September Yambolap-Tabletten. Die ganze Behandlung hatte keinen Erfolg, die Schmerzen wurden unerträglich, deshalb wurde Frau J. in die Orthopädische Klinik eingewiesen, wo festgestellt wurde, daß mehrere Wirbel verletzt waren. In der Klinik bekam die Patientin Koreberon-Tabletten und Miacalcic-Injektionen. Letztere mußten abgesetzt werden, weil der Patientin davon schlecht wurde. Die Patientin begann Ende November 1986 mit der Kalktherapie, war nach zwei Wochen schmerzfrei und fühlt sich auch heute noch wohl.

P. Gy. und seine Ehefrau waren das Opfer eines schweren Autounfalls geworden.
- Diagnose P. Gy.:: Fractura acetabuli comminut. 1. dextricum luxatio femorisdextri. Repositio, schraubenförmige Osteosynthese.
- Diagnose der Ehefrau:: Fractura acetabuli lateris dextri disloc.

Das Ehepaar verbrachte zwei Monate im Krankenhaus, nach der Entlassung wurden beide Patienten zweimal wöchentlich mit dem Krankenwagen zur Nachbehandlung gefahren. Der Unfall war am 14. September 1985 geschehen. Im April 1986 begann - vor allem im Falle der Frau - die schwache Knochenbildung Sorgen zu machen, die Befunde waren nicht günstig. Die sechs Wochen nach Beginn der Kalkpulverkur angefertigten Röntgenaufnahmen verblüfften die (von der Kalkpulverkur nichts wissenden) Ärzte gründlich. Die Belastbarkeit der Beine der Kranken wuchs langsam und stetig, heute gehen beide ohne Stock.

## Patentansprüche (Patentansprüche für folgende(n) Vertragsstaat(en): AT, BE, CH, DE, FR, GB, IT, LI, LU, NL, SE)

1. Mittel zur Behandlung von Knochenschwund, dadurch **gekennzeichnet,** daß es in feinverteilter Form
20-45 % Kalksteinmehl,
10-20 % Dolomitmehl,
30-60 % Calciumhydrogenphosphat,
gegebenenfalls höchstens 6 % Mikroelementprämix und
gegebenenfalls höchstens 5 % an sich bekannte Zusätze enthält.

2. Mittel nach Anspruch 1, dadurch **gekennzeichnet,** daß seine Teilchengröße zu 90 % unter 5 Mikron liegt.

3. Mittel nach Anspruch 1, dadurch gekennzeichnet, daß es einen Fe, Cu, Mn, Zn, J, Se, B und gegebenenfalls weitere Spurenelemente enthaltenden Mikroelementprämix enthält.

4. Mittel zum Vorbeugen gegen Knochenschwund, dadurch **gekennzeichnet,** daß es
40-60 % Kalksteinmehl,
40-60 % Dolomitmehl und gegebenenfalls max.
6 % Mikroelementprämix
enthält.

5. Mittel nach Anspruch 4, dadurch gekennzeichnet, daß seine Teilchengröße zu 90 % unter 5 Mikron liegt.

6. Mittel nach Anspruch 4, dadurch gekennzeichnet, daß es einen Fe, Cu, Mn, Zn, J, Se, B und gegebenenfalls weitere Spurenelemente enthaltenden Mikroelementprämix enthält.

## Patentansprüche (Patentansprüche für folgende(n) Vertragsstaat(en): ES, GR)

1. Verfahren zur herstellung eines Mittels zur Behandlung von Knochenschwund, dadurch **gekennzeichnet,** dass man in feinverteilter Form 20 bis 45 % Kalksteinmehl, 10 bis 20 % Dolomitmehl, 30 bis 60 %
Kalziumhydrogenphoshat, gegebenenfalls höchstens 6 %
Mikroelementprämix und gegebenenfalls höchstens 5 % von an sich bekannten Zusätzen vermengt.

2. Verfahren nach Anspruch 1, dadurch **gekennzeichnet,** dass die Teilchengrösse des Mittels zu 90 % unter 5 Mikron liegt.

3. Verfahren nach Anspruch 1, dadurch **gekennzeichnet,** dass das Mittel einen Fe, Cu, Mn, Zn, J, Se, B und gegebenenfalls weitere Spurenelemente enthaltenden Mikroelementprämix enthält.

4. Verfharen zur Herstellung eines Mittels zum Vorbeugen gegen Knochenschwund, dadurch **gekennzeichnet,** dass man 40 bis 60 % Kalksteinmehl, 40 bis 60 % Dolomitmehl und
gegebenenfalls maximal 6 % Mikroelementprämix vermengt.

5. Verfahren nach Anspruch 4, dadurch **gekennzeichnet,** dass die Teilchengrösse des Mittels zu 90 % unter 5 Mikron liegt.

6. Verfahren nach Anspruch 4, dadurch **gekennzeichnet,** dass das Mittel einen Fe, Cu, Mn, Zn, J, Se, B und gegebenenfalls weitere Spurenelemente enthaltenden Mikroelementprämix enthält.

## Claims (Claims for the following Contracting State(s): AT, BE, CH, DE, FR, GB, IT, LI, LU, NL, SE)

1. A composition for the treatment of osteoporosis, characterized by containing, in finely dispersed form,
20 to 45 % by weight of limestone powder;
10 to 20 % by weight of dolomite powder;
30 to 60 % by weight of calcium hydrogen phosphate;
optionally up to 6 % of a premix of microelements, and
optionally up to 5 % of one or more known additive(s).

2. The composition as claimed in claim 1, characterized by that the granule size of 90 % of the composition is under 5 micron.

3. The composition as claimed in claim 1, characterized by containing a premix comprising Fe, Cu Mn, Zn, J, Se, B and optionally additional microelements.

4. A composition for the prevention of osteoporosis, characterized by containing, in finely dispersed form,
40 to 60 % by weight of limestone powder,
40 to 60 % by weight of dolomite powder, and
optionally up to 6 % of a premix of microelements.

5. The composition as claimed in claim 4, characterized by that the granule size of 90 % of the composition is under 5 micron.

6. The composition as claimed in claim 4, characterized by containinga premix comprising Fe, Cu, Mn, Zn, J, Se, B and optionally additional microelement.

## Claims (Claims for the following Contracting State(s): ES, GR)

1. A process for the preparation of a composition suitable for the treatment of osteoporosis, characterized by mixing together, in finely dispersed form,
20 to 45 % by weight of limestone powder;
10 to 20 % by weight of dolomite powder;
30 to 60 % by weight of calcium hydrogen phosphate;
optionally up to 6 % of a premix of microelements, and
optionally up to 5 % of one or more known additive(s).

2. The process as claimed in claim 1, characterized by that the granule size of 90 % of the composition is under 5 micron.

3. The process as claimed in claim 1, characterized by using a premix comprising Fe, Cu Mn, Zn, J, Se, B and optionally additional microelements.

4. A process for the preparation of a composition suitable for the prevention of osteoporosis, characterized by containing, in finely dispersed form,
40 to 60 % by weight of limestone powder,
40 to 60 % by weight of dolomite powder, and
optionally up to 6 % of a premix of microelements.

5. The process as claimed in claim 4, characterized by that the granule size of 90 % of the composition is under 5 micron.

6. The process as claimed in claim 4, characterized by using a premix comprising Fe, Cu, Mn, Zn, J, Se, B and optionally additional microelement.

## Revendications (Revendications pour l'(les) Etat(s) contractant(s) suivant(s): AT, BE, CH, DE, FR, GB, IT, LI, LU, NL, SE)

1. Produit pour le traitement de l'ostéoporose caractérisé par:
- 20 - 45 % calcaire en poudre
- 10 - 20 % dolomite en poudre
- 30 - 60 % phosphate monocalcique monoacide
le cas échéant 5 % maximum de produit d'addition.

2. Le produit détaillé dans le premier point se caractérise par le fait que 90 % de ses particules mesurent moins de 5 microns.

3. Le produit détaillé dans le premier point se caractérise par le fait qu'il contient : Fe,Cu,Mn,Zn,J,Se et B et que dans le cas souhaité, il contient une mixtion de micro-éléments incluant les oligoéléments complémentaires.

4. Produit pour la prévention de l'ostéoporose, caractérisé par:
- 40 - 60 % calcaire en poudre
- 40 - 60 % dolomite en poudre
le cas échéant 6 % maximum de mixtion de micro-éléments et le cas échéant 5 % maximum de produit d'addition.

5. Le produit détaillé dans le 4ème point se caractérise par le fait que la taille de 90 % de ses particules est inférieure à 5 microns.

6. Le produit détaillé dans le 4ème point se caractérise par le fait qu'il contient: Fe,Cu,Mn,Zn,J,Se et B et que dans le cas souhaité, il contient une mixtion de micro-éléments incluant les oligo-éléments complémentaires.

## Revendications (Revendications pour l'(les) Etat(s) contractant(s) suivant(s): ES, GR)

1. Procédé pour la préparation du produit utilisable dans le traitement de l'ostéoporose, caractérisé par:
- 20 - 45 % calcaire en poudre
- 10 - 20 % dolomite en poudre
- 30 - 60 % phosphate monocalcique monoacide
le cas échéant 6 % maximum de mixtion de micro-éléments et le cas échéant 5 % maximum de produit d'addition que nous mélangeons de manière homogène.

2. Le procédé indiqué dans le premier point se caractérise par le fait que 90 % de ses particules mesurent moins de 5 microns.

3. Le procédé indiqué dans le premier point se caractérise par le fait qu'il contient: Fe,Cu,Mn,Zn,J,Se et B et que dans le cas souhaité, il contient une mixtion de micro-éléments incluant les oligo-éléments complémentaires.

4. Le procédé pour la preparation du produit pour la prévention de l'ostéoporose caractérisé par
- 40 - 60 % calcaire en poudre
- 40 - 60 % dolomite en poudre
le cas échéant 6 % maximum de mixtion de micro-éléments que nous mélangeons.

5. Le procédé selon revendication 4 caractérisé par le fait que la taille de 90 % de ces particules est inférieur à 5 microns.

6. Le procédé selon la revendication 4 caractérisé par le fait que le produit contient Fe, Cu, Mn, Zn, J, Se, B et que dans le cas souhaité, il contient une mixtion de micro-éléments incluant les oligo-éléments complémentaires.
